# EUROPEAN PATENT SPECIFICATION

(11) **EP 1 912 597 B1**
(45) Date of publication and mention of the grant of the patent: **05.05.2010**
(21) Application number: 06755882.5
(22) Date of filing: 16.05.2006
(51) Int. Cl.: A61F 2/46, B01F 11/00, B01F 13/00

(54) **CARTRIDGE FOR STERILE MIXING OF A TWO-PHASE COMPOUND, PARTICULARLY FOR TWO-COMPONENT ACRYLIC RESINS**
KARTUSCHE ZUM STERILEN MISCHEN EINER ZWEIPHASEN-VERBINDUNG, INSBESONDERE FÜR ZWEI-KOMPONENTEN-ACRYLHARZE
CARTOUCHE DESTINÉE AU MÉLANGE STÉRILE D'UN COMPOSÉ À DEUX PHASES, EN PARTICULIER DES RÉSINES ACRYLIQUES À DEUX COMPOSANTS

(30) Priority: 28.06.2005 IT VI20050187
(43) Date of publication of application: 23.04.2008
(73) Proprietor: Tecres S.P.A., 37066 Sommacampagna (VR) (IT)
(72) Inventor: FACCIOLI, Giovanni, I-46040 Monzambano (IT); SOFFIATTI, Renzo, I-37054 Nogara (IT)
(74) Representative: Feltrinelli, Secondo Andrea
(86) International application number: PCT/IB2006/001275
(87) International publication number: WO 2007/000631

(56) References cited:
- WO-A-97/18031
- WO-A-99/37256
- US-A- 5 934 803
- US-A- 6 017 349
- US-A- 6 024 480
- US-A1- 2003 021 180
- US-A1- 2004 066 706

## Description

### Technical Field

The present invention finds its application in the field of devices and methods for physical and chemical mixing of products and refers particularly to a cartridge for sterile mixing of a two-phase compound.

### Background Art

As is known, in arthroplasty operations, performed to treat bone or vertebra pathologies, and in operations for the implanting and stabilisation of bone prostheses, acrylic resins or bone cements are usually used to be introduced in the specific area to be treated.

The materials normally used in this field of surgery consist of a liquid phase, generally monomeric, used as a solvent for the polymerisation of a resin in powder form, to which may be added antibiotic drugs, promoters of growth or the like.

For these operations, the resin must be prepared directly in the operating theatre.

Consequently, the two phases are initially enclosed in two separate containers and then mixed immediately before introduction into the bone or vertebra area to be treated.

Considering the critical nature of these types of operations, it is most important that the utmost sterility of the resin and the resin dispensing devices be guaranteed at all stages.

Normally, the liquid is kept inside a plastic bag or a glass phial and then poured into a container in which powder has been previously collected. Subsequently, an operator mixes the two components using a spatula driven manually or mechanically. Finally, the compound thus obtained is introduced into a dispensing syringe and then injected under pressure through a special needle, into the bone cavity of the implant.

Such known solutions have the evident and recognised disadvantage of placing the compound into contact with the outside environment, thereby negatively affecting the sterility of the operation and making the resin a hazardous vehicle of infections for the person undergoing therapy. At the same time, the operator is placed in contact with a highly-reactive and toxic monomeric liquid, the vapours of which can freely spread in the work environment, with high risk of inhalation by the operator.

The preparation and the final composition of the mixture is, furthermore, strongly dependent on the particular skill of the operator, and so the risk exists of obtaining cements that are not perfectly homogeneous or, again, with incorrect proportions between the two phases.

In an attempt to overcome the above disadvantages, various solutions have been placed at disposal whereby one or more of such disadvantages are overcome.

From US-A-5435645, in the name of the same applicant, a device is known for mixing bone cements in which the preparation of the cement is carried out in conditions of sterility and safety for the operator. The liquid is in fact initially placed inside a first chamber and then forced to pass into a second chamber containing the powder. This way a cement is also obtained that has the right proportions between monomer and powder.

A drawback of such solution is however represented by the fact that the mixing of the two phases is done by manually shaking the whole device. This operation thus strongly depends on the skill of the single mixing operator. Being naturally impossible to precisely establish the shaking time and energy required to obtain a uniform component mix, it follows that the compound is not always shaken enough and this does not therefore show the most suitable physical characteristics. The operation is also not at all easy.

From WO-A-0183094 a device is known for mixing a bone cement in which the mixture of liquid and solid is favoured by the sliding of an agitator disc inside the mixing chamber. This way, a uniform compound is produced of correct phase proportions. Nevertheless, an evident disadvantage of such solution is represented by the fact that the liquid phase is initially taken from a container by means of a common syringe and then introduced into the mixing chamber. These phases therefore do not guarantee absolute sterility of the cement besides being inconvenient and dangerous for the operator.

From documents WO 97/18031, US 5 934 803, US 6 017 349 A,WO 99/37256 A, US 2003/021180 A1, US 2004/066706 A1, and US 6 024 480 A there are known other solutions and devices for mixing together a liquid and a solid component or phase, so as to obtain a two-phase compound, in which in particular the two phases are mixed by suitable mixing means, operable manually from the outside and provided either for moving axially inside the device or to rotate about an axis.

However even these known devices have not totally exempt from drawbacks, and in particular they would request to have a more compact construction, as well as a use involving simpler manual operations and a more practical handling.

In particular, at least for some of them, it would be desirable that the user have not to handle and use auxiliary tools, other than the device itself, for performing the mixing and dispensing steps of the compound, and/or even to remove from the mixing device the container for one of the basic phases, namely the liquid phase, once such container has been used and emptied of the liquid phase contained therein.

### Disclosure of the Invention

The purpose of this invention is to overcome the above drawbacks and make a cartridge for mixing a two-phase compound with clearly efficient features and which is relatively inexpensive.

A particular purpose is to make a cartridge for mixing a two-phase compound that permits obtaining a compound with homogeneous chemical, physical and mechanical characteristics in conditions of absolute sterility.

A further purpose is to make a cartridge for mixing a two-phase compound which is easy and safe to use for each operator.

Such purposes, as well as others which will appear clearer later on, are achieved by a cartridge for the sterile mixing of a two-phase compound, as in claim 1, comprising a first tubular body defining a first collection chamber substantially longitudinal for a solid phase, a second tubular body defining a second collection chamber for a liquid phase, means for mixing the liquid phase with the solid phase, characterized by the fact that the mixing means comprise agitator means acting on the mixture inside the first chamber with the first tubular body in substantially stationary conditions.

Thanks to this particular configuration, the cartridge according to the invention favours the dispersion of the solid phase inside the liquid phase thus making it possible to obtain a compound with uniform chemical, physical and mechanical properties in conditions of absolute sterility. The presence of agitator means in fact permits the uniform diffusion of the solid phase in the liquid phase, thereby ensuring perfect component mixing homogeneousness.

Advantageously, the agitator means can include a mobile agitator element which will be housed inside the first chamber and can be at least partially hollow and will be preferably transversal and shaped like a grid.

Preferably, the mobile agitator element can be coupled to means of movement that can be operated by an operator.

Advantageously, the means of movement can include a gripping element outside the first and second chamber and rigidly coupled to the mobile agitator element by means of suitable linking means. The latter may, in turn, comprise at least one, preferably a pair of rods with a first end connected to the gripping element and a second end connected to the mobile agitator element.

Preferably, the first tubular body can feature a top cover with at least a first guide opening for the connection rod which can be shaped like a slot.

Thanks to this characteristic, it will be possible to make a cartridge for mixing a two-phase compound that is easy and safe to use by each operator. The agitator element will in fact be of simple and light manufacture and may, furthermore, be easily operated by means of the alternate movement of the grip element and with minimum expenditure of energy. Moreover, the characteristics of the compound will always be reproducible to the same extent.

As required, the means for transferring the liquid phase into the first chamber can comprise at least one through cavity made on the end portion of the second tubular body.

Preferably, the transfer means can comprise pressure means operating between the first and the second chamber and, according to a particular form of embodiment, the pressure means can feature an open portion of the side wall of the second tubular body enclosed by an elastically yielding membrane deformable towards the inside.

Thanks to this latter characteristic, during mixing, the compound components are prevented from coming into contact with the outside and sterility is maintained.

### Brief description of the drawings

Further characteristics and advantages of the invention will appear even more evident from the detailed description of a preferred, but not exclusive, form of embodiment of a mixing cartridge according to this invention, illustrated by way of non limiting example in the attached drawings, wherein:
**FIG. 1** is a front view of a cartridge according to the invention in a first preferred embodiment;
**FIG.2** is an exploded view of cartridge of FIG.1;
**FIG.3** is an enlarged view of a detail of FIG.2;
**FIG.4** is a view from above of a first detail of FIG.1;
**FIG.5** is a section view according to the tracing plan ***I-I*** of a further detail of the cartridge in FIG.1;
**FIG.6** is a front view of a cartridge according to the invention in a second preferred form of embodiment.

### Ways of carrying out the invention

With reference to the above mentioned figures, the cartridge according to the invention, generally designated by reference numeral 1, may be used to mix, in sterile conditions, the components of a bone cement acrylic resin for arthroplasty operations or bone or joint prostheses implants. The compound will consist of a liquid phase, generally monomeric, and of a solid phase in powder state, if necessary with the addition of antibiotic agents or growth promoters, which polymerises once dissolved in the liquid phase. According to another possible use, the compound may also be a pharmaceutical product chosen from among the antibiotics, vitamins or the like. The two phases will, in any case, be initially kept separate.

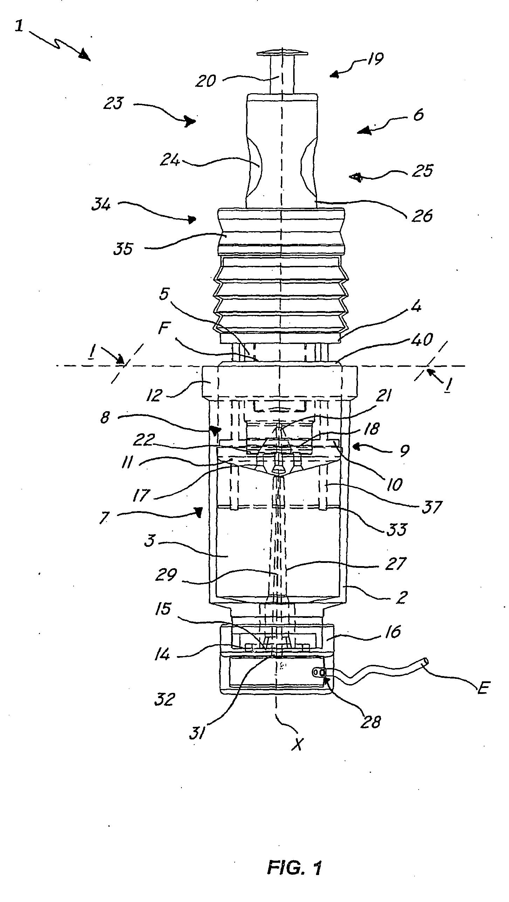

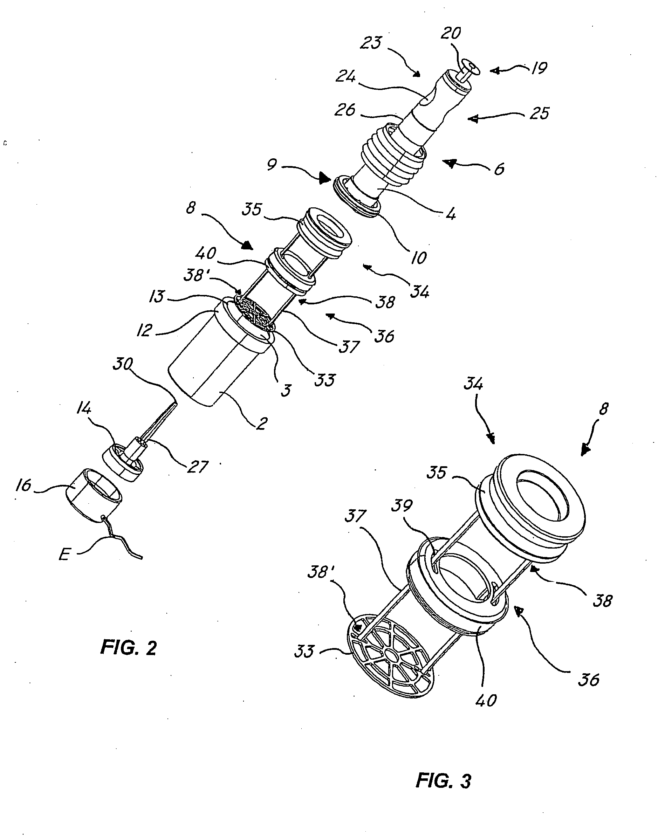

As shown particularly in Figures 1 and 2, the cartridge comprises a first tubular body **2,** defining a first collection chamber **3** substantially longitudinal for the solid phase, and a second tubular body **4**, defining a second collection chamber **5** for the liquid phase. The sterile transit of the liquid phase from the second chamber **5** into the first chamber **3** is by means of suitable transfer means **6** which maintain sterility.

The different parts of the cartridge **1** can be made of plastic, rigid or semi-rigid, with suitable stress resistance characteristics.

Preferably the material will be transparent and the cartridge **1** will be of the disposable type.

According to the invention, means **7** are comprised for mixing the liquid phase with the solid phase which comprise further agitator means **8** to favour the dispersion of the solid phase inside the liquid phase while keeping the first tubular body **2** substantially stationary.

As illustrated, the second tubular body **4** features the lower end portion **9** housed sliding and coaxially inside the first tubular body **2.**

Suitably, at end portion **9** is a ring-shaped flange **10** with outer diameter **d1** slightly less than the diameter **d2** of the first chamber **3.** The flange **10,** together with the end portion **9,** will act as a piston **11** in the last resin dispensing phase.

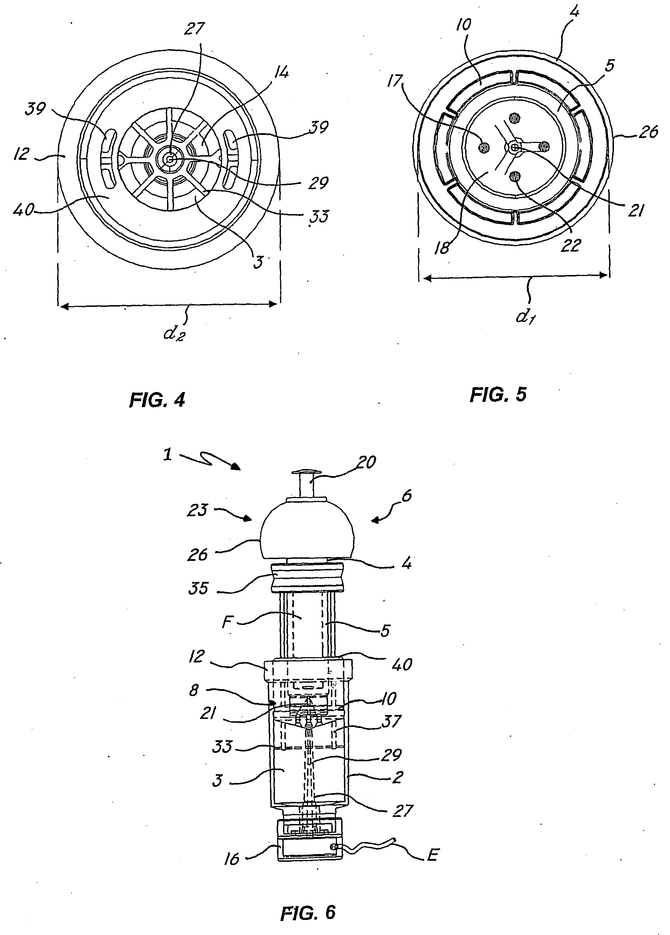

As specifically shown in Figure **4****,** the first tubular body **2** will feature an upper portion **12** with an entry door **13** made in central position to allow transit of the second tubular body **4.** The first body **2** will on the other hand be closed in opposite position by a rear wall **14** featuring a through hole **15** for dispensing the mixed resin towards suitable external implantation means **E.** During the mixing stage, the through hole **15** will be closed by a closing element **16,** such as, for example, a cap that screws onto it, which will be removed when the compound is dispensed ready for use.

Advantageously, the transfer means **6** for putting the collection chambers **3, 5** in fluid communication will comprise a series of through cavities **17** made on the lower **wall 18** of the second body **4,** visible in Figure 5.

The liquid phase can be introduced into the second chamber **5** inside a suitable container **F,** such as for instance a breakable glass phial, through specific breaking means **19** positioned inside the second chamber **5.** The means **19** will preferably comprise an upper cylindrical element **20** sliding inside the second chamber **5,** and a pointed element **21** longitudinally opposite. The upper element **20** will be operated from outside so as to push the phial **F** against the pointed element **21** causing this to break and, therefore, the liquid phase to pour into the first chamber **3** passing through the through cavities **17.** Downstream of the latter, a first filter element **22** will also be located to prevent the transit of fragments of glass produced by the breakage of phial **F** or, again, the transit of the solid phase in the opposite direction.

Preferably, to favour the transit of the liquid phase inside the first chamber **3,** the transfer means **6** can comprise pressure means **23** able to determine, inside the first chamber **3,** a reduction in pressure and consequently a lower pressure compared to that existing inside the second chamber **5.** This way, the liquid will be recalled inside the first chamber **3** by the vacuum generated inside this.

In a first preferred but not exclusive form of embodiment of the invention, shown in figures 1 and 2, the pressure means **23** will comprise an elastically yielding membrane **24** which closes an open portion **25** of the side wall **26** of the second tubular body **4.** By adjusting the pressure on membrane **24,** the operator can change the volume inside the second collection chamber **5** and when this is released, the above vacuum will be produced.

In a second form of embodiment, shown in fig. 6: the pressure means **23** can consist of the same piston **11** which, operated by the alternative sliding in axial direction of the second tubular body **4,** will determine the vacuum inside the first collection chamber **3** and, consequently, the transit of the liquid inside this.

In each configuration, a stop element **27** will also be fitted to restrict the movement of the piston **11.** The element **27** will be substantially longitudinal, and will protrude inside the first chamber **3** and will be associated with the closing element **16.** This way, any contact between piston **11** and the powder will be avoided during generation of the vacuum in the first chamber **3.**

In order to achieve a strong vacuum inside the first chamber **3,** the transfer means **6** will comprise suitable fluid connecting means **28** of the first collection chamber **3** to the external vacuum means **E.** The connecting means **28** will comprise a pipe **29** made inside the stop element **27** and having a longitudinal direction **X**, according to the development of element **27** itself. The pipe **29** will feature a free entrance **30** inside the first chamber **3** and an exit **31** inside the closing element **16** and downstream of which a second filter element **32** will be positioned. The latter can be a microbiological filter, for example of the active charcoal type, designed to preserve the sterility of the compound housed in the first collection chamber **3,** particularly during the vacuum creation phase. After the transit of the liquid phase inside the first chamber **3,** the mixing will occur of the two phases present at the same time inside the first chamber **3,** manually operating the agitator means **8,** particularly shown in Fig. **3****.**

Advantageously, the means **8** will comprise a mobile agitator element **33** inside the first chamber **3** and at least partially hollow. Preferably, the element **33** will be configured like a flat grid with development substantially transversal with respect to the longitudinal dimension of the cartridge **1,** its being possible furthermore to make it of the same material as cartridge **1** or of a similar material.

The movement of the agitator element **33** will be suitably simplified by coupling this with suitable means of movement **34** that can be operated from outside by an operator.

The means **34** will comprise a gripping element **35** outside the collection chamber of phases **3, 5** configured like a round crown coaxial to the second tubular body **4,** rigidly coupled to the agitator element **33** by means of specific linking means **36.** The latter will be substantially a pair of rods **37** arranged symmetrically to the development axis **X.**

The rods **37** will have a first end **38** connected to the gripping element **35** and a second end **38'** connected to the agitator element **33.** Furthermore, the rods **37** will be conducted through respective guide openings **39** made in a ring nut **40** that can be fitted at the upper portion **12** of the first tubular body **2,** so as to slide sealed. The guide openings **39** will be configured as slots to permit partial rotation of the agitator means **8** around the longitudinal direction **X,** so as to ensure more efficient mixing of the phases. After mixing, the resin will be ready to be dispensed towards the external implantation means. For this purpose, the closing element **16** will be removed from the hole **15** on the rear wall **14,** the implantation means **E** will be connected and the resin will be dispensed by means of adequate pressure applied by means of piston **11** operated by means of the thrust applied by the operator on the second tubular body **4.**

From the above description, it is evident that the cartridge according to the invention achieves the intended purposes, and particularly to allow the phase mixing so as to obtain a two-phase compound with homogenuous chemical, physical and mechanical characteristics and in conditions of absolute sterility.

Furthermore, thanks to the special configuration of the mixing means, it is possible to achieve a cartridge being safe and easy to use for any operator.

The cartridge according to the invention is susceptible of numerous modifications and variations, all of which falling within the scope of the inventive concept as contained in the enclosed claims. All the details can be replaced with others that are technically equivalent and the materials used may be any according to requirements without because of this moving outside the protection scope of the invention.

The cartridge has also been described with special reference to the attached figures, the reference numbers used in the description and claims are used to upgrade the intelligence of the invention and do not represent any limitation to the claimed protection scope.

## Claims

1. A cartridge for the sterile mixing of a two-phase compound, particularly an acrylic resin, consisting of a liquid phase and a solid phase which can be mixed immediately before dispensing, comprising:
- a first tubular body (2) defining a first collection chamber (3) for a solid phase, said first tubular body (2) extending substantially longitudinal along a longitudinal axis (X),
- a second tubular body (4) defining a second collection chamber (5) for a liquid phase, said second tubular body (4) being arranged for sliding coaxially to and inside said first tubular body (2),
- transfer means (6) of said liquid phase from said second (5) to said first chamber (3), and
- means for mixing (7) said liquid phase with said solid phase,
wherein said means for mixing (7) comprise agitator means (8) acting on the mixture of said phases inside said first chamber (3) with said first tubular body (2) in substantially stationary conditions, so as to favour the dispersion of the solid phase inside the liquid phase thus obtaining a compound with uniform physical and mechanical properties in conditions of absolute sterility,
said cartridge being **characterized in that** said agitator means (8) comprise an agitator body (33, 36, 37) which is arranged for moving coaxially to and between said first tubular body (2) and said second tubular body (4), said agitator body (33, 36, 37) being substantially hollow for housing and receiving axially said second tubular body (4),
said agitator means (8) comprising a mobile agitator element (33) integral with said agitator body and housed inside said first chamber (3),
said agitator means (8) being coupled to means of movement (34), comprising a gripping element (35) arranged outside said first (3) and said second (5) chamber, which means of movement are suitable to be operated by an operator for moving said mobile agitator element (33) in said first chamber (3), and
said agitator body of said agitator means (8) including suitable linking means (36), spaced from said longitudinal axis (X), which are provided for rigidly coupling said gripping element (35) to said mobile agitator element (33).

2. Cartridge according to claim 1, **characterized by** the fact that said agitator element (33) is at least partially hollow and with a development substantially transversal with respect to the longitudinal dimension of said first tubular body (2).

3. Cartridge according to claim 1, **characterized by** the fact that said mobile agitator element (33) is substantially shaped like a grid.

4. Cartridge according to the preceding claim, **characterized by** the fact that said linking means (36) comprise at least one, preferably a pair of rods (37) with a first end (38) connected to said gripping element (35) and a second end (38') connected to said mobile agitator element (33).

5. Cartridge according to the preceding claim, **characterized by** the fact that said first tubular body (2) features an upper portion (12) with an entry door (13) for said second tubular body (4) and a ring nut (40) with at least one guide opening (39) for said at least one rod (37), said first body (2) also featuring a rear wall (14) with a through hole (15) for dispensing the mixed compound and a removable closing element (16) for shutting said through hole (15).

6. Cartridge according to the preceding claim, **characterized by** the fact that said at least one guide opening (39) is configured as a slot to permit at least a partial rotation of said agitator means (8) around the longitudinal development direction (X) of said first tubular body (2).

7. Cartridge according to the preceding claim, **characterized by** the fact that said second tubular body (4) features an end portion (9) housed sliding and coaxially inside said first tubular body (2) passing through said entry door (13) of said upper portion (12).

8. Cartridge according to the preceding claim, **characterized by** the fact that said end portion (9) of said second body (4) features a ring-shaped flange (10) with outer diameter (dl) corresponding to diameter (d2) of said first chamber (3) to define a piston (11) for dispensing the mixed compound.

9. Cartridge according to the preceding claim, **characterized by** the fact that said transfer means (6) of said liquid phase from said second (5) to said first chamber (3) comprise at least one through cavity (17) made by said end portion (9) of said second tubular body (4), said at least one through cavity (17) being fitted with at least one first filter element (22).

10. Cartridge according to claim 1, **characterized by** the fact that said transfer means (6) comprise pressure means (23) acting between said first (3) and said second chamber (5).

11. Cartridge according to the preceding claim, **characterized by** the fact that said pressure means (23) comprise an open portion (25) of the side wall (26) of said second tubular body (4), closed by an elastically yielding membrane (24) deformable towards the inside.

12. Cartridge according to claim 1, **characterized by** the fact that it comprises a stop element (27) for said agitator element (33) substantially longitudinal and associated with said closing element (16) and protruding inside said first chamber (3).

13. Cartridge according to claim 1 **characterized by** the fact that said transfer means (6) comprise fluid connecting means (28) of said first collection chamber (3) to the external vacuum means (E) so as to increase the vacuum inside said first chamber (3).

14. Cartridge according to claim 1, **characterized by** the fact that said fluid connecting means (28) comprise a substantially longitudinal pipe (29) made inside said stop element (27), said pipe (29) having an entrance (30) inside said first chamber and an exit (31) inside said closing element (16), said closing element (16) featuring a second filter element (32) downstream said exit (31).

## Patentansprüche

1. Kartusche zum sterilen Anmischen einer Zwei-Phasen-Masse, insbesondere eines Acrylharzes, die aus einer Flüssigphase und einer Festphase besteht, die unmittelbar vor dem Ausgabe vermischt werden können, umfassend:
- einen ersten rohrförmigen Körper (2), der eine erste Sammelkammer (3) für eine Festphase definiert, wobei sich dieser erste rohrförmige Körper (2) im Wesentlichen in Längsrichtung längs einer Längsachse (X) erstreckt,
- einen zweiten rohrförmigen Körper (4), der eine zweite Sammelkammer (5) für eine Flüssigphase definiert, wobei dieser zweite rohrförmige Körper (4) dazu bestimmt ist, koaxial zum und innerhalb des ersten rohrförmigen Körpers (2) zu gleiten,
- Mittel zum Übertragen (6) der Flüssigphase von der zweiten (5) zur ersten Kammer (3), und
- Mittel zum Mischen (7) der Flüssigphase mit der Festphase,
wobei die Mittel zum Mischen (7) Rührmittel (8) umfassen, die auf das Gemisch der Phasen in der ersten Kammer (3) einwirken, während sich der erste rohrförmige Körper (2) in einem im Wesentlichen stationären Zustand befindet, um die Verteilung der Festphase in der Flüssigphase zu begünstigen, so dass eine Masse mit homogenen physikalischen und mechanischen Eigenschaften unter absolut sterilen Bedingungen erhalten wird,
wobei die Kartusche **dadurch gekennzeichnet ist, dass** die Rührmittel (8) einen Rührkörper (33, 36, 37) umfassen, der dazu bestimmt ist, sich koaxial zu und zwischen dem ersten rohrförmigen Körper (2) und dem zweiten rohrförmigen Körper (4) zu bewegen, wobei der Rührkörper (33, 36, 37) im Wesentlichen hohl ist, um den zweiten rohrförmigen Körper (4) axial aufzunehmen und zu empfangen,
wobei die Rührmittel (8) ein bewegliches Rührelement (33) umfassen, das mit dem Rührkörper ein Ganzes bildet und in der ersten Kammer (3) untergebracht ist,
wobei die Rührmittel (8) mit Bewegungsmitteln (34) verbunden sind, die ein Greifelement (35) umfassen, das außerhalb der ersten (3) und der zweiten (5) Kammer angeordnet ist, wobei die Bewegungsmittel dazu geeignet sind, von einem Anwender betätigt zu werden, um das bewegliche Rührelement (33) in der ersten Kammer (3) zu bewegen, und
wobei der Rührkörper der Rührmittel (8) geeignete, von der Längsachse (X) beabstandete Verbindungsmittel (36) einschließt, die dazu vorgesehen sind, das Greifelement (35) starr mit dem beweglichen Rührelement (33) zu verbinden.

2. Kartusche nach Anspruch 1, **dadurch gekennzeichnet, dass** das Rührelement (33) zumindest teilweise hohl ist und sich im Wesentlichen quer zur Längserstreckung des ersten rohrförmigen Körpers (2) ausdehnt.

3. Kartusche nach Anspruch 1, **dadurch gekennzeichnet, dass** das bewegliche Rührelement (33) im Wesentlichen wie ein Gitter geformt ist.

4. Kartusche nach dem vorherigen Anspruch, **dadurch gekennzeichnet, dass** die Verbindungsmittel (36) mindestens einen und vorzugsweise ein Paar von Stäben (37) mit einem mit dem Greifelement (35) verbundenen ersten Ende (38) und einem mit dem beweglichen Rührelement (33) verbundenen zweiten Ende (38') umfassen.

5. Kartusche nach dem vorherigen Anspruch, **dadurch gekennzeichnet, dass** der erste rohrförmige Körper (2) einen oberen Abschnitt (12) mit einer Eintrittsöffnung (13) für den zweiten rohrförmigen Körper (4) und einem Schraubring (40) mit mindestens einer Führungsöffnung (39) für den mindestens einen Stab (37) aufweist, wobei der erste Körper (2) außerdem eine Rückwand (14) mit einem Durchgangsloch (15) zum Ausgeben der gemischten Masse und ein entfernbares Verschlusselement (16) zum Verschließen dieses Durchgangslochs (15) aufweist.

6. Kartusche nach dem vorherigen Anspruch, **dadurch gekennzeichnet, dass** die mindestens eine Führungsöffnung (39) als Schlitz ausgebildet ist, um zumindest eine teilweise Drehung der Rührmittel (8) um die Richtung der Längsausdehnung (X) des ersten rohrförmigen Körpers (2) zu ermöglichen.

7. Kartusche nach dem vorherigen Anspruch, **dadurch gekennzeichnet, dass** der zweite rohrförmige Körper (4) einen gleitend und koaxial im ersten rohrförmigen Körper (2) untergebrachten Endabschnitt (9) aufweist, der die Eintrittsöffnung (13) des oberen Abschnitts (12) durchquert.

8. Kartusche nach dem vorherigen Anspruch, **dadurch gekennzeichnet, dass** der Endabschnitt (9) des zweiten Körpers (4) einen ringförmigen Flansch (10) mit einem dem Durchmesser (d2) der ersten Kammer (3) entsprechenden Außendurchmesser (dl) aufweist, um einen Kolben (11) zum Ausgeben der gemischten Masse zu definieren.

9. Kartusche nach dem vorherigen Anspruch, **dadurch gekennzeichnet, dass** die Mittel zum Übertragen (6) der Flüssigphase von der zweiten (5) zur ersten Kammer (3) mindestens einen aus dem Endabschnitt (9) des zweiten rohrförmigen Körpers (4) bestehenden durchgehenden Hohlraum (17) umfassen, wobei der mindestens eine durchgehende Hohlraum (17) mit mindestens einem ersten Filterelement (22) versehen ist.

10. Kartusche nach Anspruch 1, **dadurch gekennzeichnet, dass** die Übertragungsmittel (6) Druckmittel (23) umfassen, die zwischen der ersten (3) und der zweiten Kammer (5) wirken.

11. Kartusche nach dem vorherigen Anspruch, **dadurch gekennzeichnet, dass** die Druckmittel (23) einen offenen Abschnitt (25) der Seitenwand (26) des zweiten rohrförmigen Körpers (4) umfassen, der durch eine elastisch nachgiebige Membrane (24) verschlossen wird, die nach innen verformbar ist.

12. Kartusche nach Anspruch 1, **dadurch gekennzeichnet, dass** sie ein Anschlagelement (27) für das Rührelement (33) umfasst, das im Wesentlichen längslaufend und mit dem Verschlusselement (16) verbunden ist und in die erste Kammer (3) hineinragt.

13. Kartusche nach Anspruch 1, **dadurch gekennzeichnet, dass** die Übertragungsmittel (6) Mittel für die Fluidverbindung (28) der ersten Sammelkammer (3) mit äußeren Vakuummitteln (E) umfassen, um das Vakuum in der ersten Kammer (3) zu erhöhen.

14. Kartusche nach Anspruch 1, **dadurch gekennzeichnet, dass** die Fluidverbindungsmittel (28) ein im Wesentlichen längslaufendes Rohr (29) umfassen, das im Anschlagelement (27) ausgebildet ist, wobei das Rohr (29) einen Eingang (30) innerhalb der ersten Kammer und einen Ausgang (31) innerhalb des Verschlusselements (16) aufweist, wobei das Verschlusselement (16) ein zweites Filterelement (32) abwärts des Ausgangs (31) aufweist.

## Revendications

1. Cartouche pour le mélange stérile d'un composé à deux phases, en particulier une résine acrylique, consistant en une phase liquide et une phase solide qui peuvent être mélangées immédiatement avant la distribution, comprenant :
- un premier corps tubulaire (2) définissant une première chambre collectrice (3) pour une phase solide, ledit premier corps tubulaire (2) s'étendant sensiblement longitudinalement suivant un axe longitudinal (X),
- un deuxième corps tubulaire (4) définissant une deuxième chambre collectrice (5) pour une phase liquide, ledit deuxième corps tubulaire (4) étant agencé pour coulisser de manière coaxiale et à l'intérieur dudit premier corps tubulaire (2) ,
- des moyens de transfert (6) de ladite phase liquide de ladite deuxième chambre (5) à ladite première chambre (3), et
- des moyens (7) pour mélanger ladite phase liquide avec ladite phase solide, dans lequel lesdits moyens de mélange (7) comprennent des moyens d'agitateur (8) agissant sur le mélange desdites phases à l'intérieur de ladite première chambre (3) avec ledit premier corps tubulaire (2) dans des conditions sensiblement immobiles, de manière à favoriser la dispersion de la phase solide dans la phase liquide en obtenant ainsi un composé avec des propriétés physiques et mécaniques uniformes dans des conditions de stérilité absolue,
ladite cartouche étant **caractérisée en ce que** lesdits moyens d'agitateur (8) comprennent un corps d'agitateur (33, 36, 37) qui est agencé pour se déplacer de manière coaxiale et entre ledit premier corps tubulaire (2) et ledit deuxième corps tubulaire (4), ledit corps d'agitateur (33, 36, 37) étant sensiblement creux pour loger et recevoir axialement ledit deuxième corps tubulaire (4),
lesdits moyens d'agitateur (8) comprenant un élément agitateur mobile (33) d'une seule pièce avec le corps d'agitateur et logé à l'intérieur de ladite première chambre (3),
lesdits moyens d'agitateur (8) étant couplés à des moyens d'entraînement (34), comprenant un élément de serrage (35) agencé à l'extérieur de ladite première chambre (3) et de ladite deuxième chambre (5), lesdits moyens d'entraînement (35) étant adaptés pour être actionnés par un opérateur pour déplacer ledit élément agitateur mobile (33) dans ladite première chambre (3), et
ledit corps d'agitateur desdits moyens d'agitateur (8) comprenant des moyens de liaison appropriés (36), espacés dudit axe longitudinal (X), qui sont prédisposés pour coupler rigidement ledit élément de serrage (35) avec ledit élément d'agitateur mobile (33).

2. Cartouche selon la revendication 1, **caractérisée en ce que** ledit élément d'agitateur (33) est au moins partiellement creux et avec une extension sensiblement transversale par rapport à la dimension longitudinale dudit premier corps tubulaire (2).

3. Cartouche selon la revendication 2, **caractérisée en ce que** ledit élément d'agitateur mobile (33) est formé sensiblement comme une grille.

4. Cartouche selon la revendication précédente, **caractérisée en ce que** lesdits moyens de liaison (36) comprennent au moins une et de préférence deux tiges (37) avec une première extrémité (38) reliée audit élément de serrage (35) et une deuxième extrémité (38') reliée audit élément d'agitateur mobile (33).

5. Cartouche selon la revendication précédente, **caractérisée en ce que** ledit premier corps tubulaire (2) présente une portion supérieure (12) avec une ouverture d'entrée (13) pour le deuxième corps tubulaire (4) et un écrou à anneau (40) avec au moins une ouverture de guidage (39) pour ladite au moins une tige (37), ledit premier corps (2) présentant également une paroi postérieure (14) avec un trou traversant (15) pour distribuer le composé mélangé et un élément de fermeture (16) pour obturer ledit trou traversant (15).

6. Cartouche selon la revendication précédente, **caractérisée en ce que** ladite au moins une ouverture de guidage (39) est configurée comme une fente pour permettre au moins une rotation partielle desdits moyens d'agitateur (8) autour de la direction d'extension longitudinale (X) dudit premier corps tubulaire (2).

7. Cartouche selon la revendication précédente, **caractérisée en ce que** ledit deuxième corps tubulaire (4) présente une portion d'extrémité (9) logée de manière coulissante et coaxiale à l'intérieur dudit premier corps tubulaire (2) en passant à travers ladite ouverture d'entrée (13) de ladite portion supérieure (12).

8. Cartouche selon la revendication précédente, **caractérisée en ce que** ladite portion d'extrémité (9) dudit deuxième corps (4) présente une bride annulaire (10) avec un diamètre extérieure (d1) correspondant au diamètre (d2) de ladite première chambre (3) pour définir un piston (11) pour distribuer le composé mélangé.

9. Cartouche selon la revendication précédente, **caractérisée en ce que** lesdits moyens de transfert (6) de ladite phase liquide de ladite deuxième chambre (5) à ladite première chambre (3) comprennent au moins une cavité traversante (17) constituée par ladite portion d'extrémité (9) dudit deuxième corps tubulaire (4), ladite au moins une cavité traversante (17) étant pourvue d'au moins un premier élément de filtre (22).

10. Cartouche selon la revendication 1, **caractérisée en ce que** lesdits moyens de transfert (6) comprennent des moyens de pression (23) agissant entre ladite première chambre (3) et ladite deuxième chambre (5).

11. Cartouche selon la revendication précédente, **caractérisée en ce que** lesdits moyens de pression (23) comprennent une portion ouverte (25) de la paroi latérale (26) dudit deuxième corps tubulaire (4), fermée par une membrane élastiquement flexible (24) déformable vers l'intérieur.

12. Cartouche selon la revendication 1, **caractérisée en ce qu'**elle comprend un élément de butée (27) pour ledit élément d'agitateur (33) sensiblement longitudinal et associé audit élément de fermeture (16) et faisant saillie à l'intérieur de ladite première chambre (3).

13. Cartouche selon la revendication 1, **caractérisée en ce que** lesdits moyens de transfert (6) comprennent des moyens de communication de fluide (28) de ladite première chambre (3) avec les moyens de vide extérieurs (E) de manière à augmenter le vide à l'intérieur de ladite première chambre (3).

14. Cartouche selon la revendication 1, **caractérisée en ce que** lesdits moyens de communication de fluide (28) comprennent un tube sensiblement longitudinal (29) réalisé à l'intérieur dudit élément de butée (27), ledit tube (29) comportant une entrée (30) à l'intérieur de ladite première chambre et une sortie (31) à l'intérieur dudit élément de fermeture (16), ledit élément de fermeture (16) présentant un deuxième élément de filtre (32) en aval de ladite sortie (31).
